# EUROPEAN PATENT SPECIFICATION

(11) **EP 1 810 649 B1**
(45) Date of publication and mention of the grant of the patent: **14.07.2010**
(21) Application number: 05793230.3
(22) Date of filing: 14.10.2005
(51) Int. Cl.: A61F 13/06

(54) **SPORTSWEAR**
SPORTBEKLEIDUNG
VETEMENT DE SPORT

(30) Priority: 19.10.2004 JP 2004304500; 18.05.2005 JP 2005145837
(43) Date of publication of application: 25.07.2007
(73) Proprietor: ONYONE Co., Ltd., Nagaoka-shi, Niigata 940-8548 (JP)
(72) Inventor: ONDA, Hironori, c/o ONYONE CO., LTD., Nagaoka-shi, Niigata 940-8548 (JP)
(74) Representative: HOFFMANN EITLE
(86) International application number: PCT/JP2005/018943
(87) International publication number: WO 2006/043476

(56) References cited:
- EP-A- 0 733 351
- JP-A- 4 050 302
- JP-A- 4 057 904
- JP-A- 9 241 906
- JP-A- 10 280 209
- JP-A- 11 012 814
- JP-A- 2001 214 303
- JP-A- 2004 003 112

## Description

### TECHNICAL FIELD

The present invention relates to a sports garment capable of preventing contusions, bruising, muscle strain, and other sports injuries when the sports garment is worn during sports activities.

### BACKGROUND ART

EP 0 733 351 A2 describes a wearing article with a strapping function for wearing on a human body in pressed relation to a surface of the human body, has a heavily-stretchable portion which has an excellent tightening force and is adapted to be held against a required portion of the human body so as to extend generally along muscle fibers over a region from a tendon to a central portion of the muscle. The remainder of the wearing article is defined by an easily-stretchable portion. With this arrangement, by merely wearing this wearing article on the required portion of the body, the heavily-stretchable portion tightens only a required portion of the body to support the central portion of the relevant muscle, thereby easily achieving a strapping function.

The present applicant has invented a sports garment whereby the same effects as taping are obtained and contusions, bruising, muscle strain, and other sports injuries are prevented through a simple method in which a taping theory in sports science is applied to actively form a part having a difference in elasticity in a sports garment (see Japanese Patent No. 2732394).

### DISCLOSURE OF THE INVENTION

### [Problems to Be Solved by the Invention]

The present invention was developed through further research based on the invention described above.

### [Means for Overcoming the Above-mentioned Problems]

A summary of the present invention will be given with reference to the accompanying drawings.

The sports garment of the present invention is characterized in that a garment body 2 for adhering to a body 1 is formed using a material that has low frictional resistance and slides easily against the skin surface of a body 1; a resistance lining part 3 is formed on the internal surface of the garment body 2 by using a material that has high frictional resistance and does not easily slide against the skin surface of a body 1; a position at which the resistance lining part 3 adheres to a skin surface of the body 1 varies less easily than that of another adhering internal surface of the garment body 2; the garment body 2 and the resistance lining part 3 are formed so as to be elastic; an elasticity of the resistance lining part 3 is set so as to be less than an elasticity of the garment body 2; and the resistance lining part 3 is formed in a direction whereby the same effects are obtained as a taping effect in which tape is wrapped onto a body 1 to restrict movement of muscles, joints, and other parts so as to prevent contusions, bruising, muscle strain, sprain, bone fracture, and other injury.

The sports garment according to a second aspect is characterized in that the resistance lining part 3 is bonded and fixed to an internal surface of the garment body 2.

### [Effect of the Invention]

Through a structure such as the one described above, the present invention provides a markedly superior sports garment capable of producing the same effects as taping, and of preventing contusions, bruising, muscle strain, and other sports injuries through a simple method in which a taping theory in sports science is applied to actively endow a sports garment with frictional resistance with respect to a body (skin surface) so as to create an environment in which muscles, joints, and other parts move in appropriate ranges.

The present invention also provides an extremely practical and innovative sports garment whereby the aforementioned actions/effects can be obtained merely by wearing the sports garment of the present invention on the body, without the need for inconvenient taping.

In the present invention, since the resistance lining part always adheres to the skin surface of the body when the garment is worn, a sports garment is provided that has even more superior practicality and demonstrates the aforementioned operations/effects extremely effectively.

In the present invention, since the resistance lining part is elastic, there is no extreme impediment to movement when the sports garment is worn, and excellent ease of movement is obtained. The resistance lining part is also elastic, but has little elasticity in relation to the garment body and does not stretch in the same manner as the garment body. A sports garment is therefore provided that reliably supports muscles and other parts, produces the aforementioned taping effects, and has excellent practicality.

In the invention according to the second aspect, a garment body having a resistance lining part can easily be designed and implemented, and the resistance lining part evenly supports muscles and other parts using a uniform pressure. Since the garment body is also sewn only in a small number of positions, it is possible to provide a sports garment that allows easier movement with no discomfort, thus making the sports garment even more practical.

### BRIEF DESCRIPTION OF THE DRAWINGS

FIG. 1 is a diagram showing a state in which Example 1 is in use;
FIG. 2 is a diagram showing a state in which Example 1 is in use during exercise;
FIG. 3 is a front view showing a state in which Example 2 is in use;
FIG. 4 is a diagram showing a state in which Example 2 is in use during exercise;
FIG. 5 is a side view showing a state in which Example 2 is in use;
FIG. 6 is a rear view showing a state in which Example 2 is in use; and
FIG. 7 is a diagram showing Example 3 from the rear.

Key to Symbols:
1: body
2: garment body
3: resistance lining part

### BEST MODE FOR CARRYING OUT THE INVENTION

Preferred embodiments of the present invention (ways of implementing the invention), and the operation of the present invention will be briefly described based on the accompanying drawings.

When the garment body 2 is worn on the body 1, the resistance lining part 3 does not easily slide against the skin surface of the body 1, due to the large frictional resistance of the resistance lining part 3, whereas the internal surfaces of the other parts of the garment body 2 have minimal frictional resistance with the body 1, and slide easily.

Accordingly, in the portion of the body 1 in contact with the resistance lining part 3, the movement of skin and the muscles/joints and other parts in the skin is restricted by the frictional resistance of the resistance lining part 3. By forming the resistance lining part 3 in the taping direction, movement of muscles/joints and other parts in this direction is restricted so as to occur in the appropriate allowable range according to taping theory, and the effects obtained are the same as the taping effects aimed at preventing contusions, bruising, muscle strain, and other sports injuries.

The resistance lining part 3 conformed (aligned in the taping direction) with the muscles also acts to move fatigued muscles in the appropriate allowable range. Therefore, muscle tension is alleviated and fatigue is reduced.

Bones that constitute joints are sometimes pulled by muscle tension, which causes joints to become misaligned and painful, but placing the resistance lining part 3 in contact with the skin to create a taping effect has the effect of alleviating muscle tension.

A massaging effect on muscles and other parts can be anticipated from the frictional movement of the resistance lining part 3 against the skin surface, thus creating a suitable environment for exercise. Increased circulation of blood and lymph, and improvement of poor circulation can also be anticipated, as well as pain relief and the activation of natural analgesic effects in the body.

Accordingly, the inconvenience of applying tape to the body 1 such as in taping is eliminated, and an innovative sports garment is provided whereby the aforementioned operations/effects on the same part of the body can be anticipated any number of times and at any time merely by wearing the garment body 2 on the body 1 in the proper manner.

The garment body 2 is preferably made into a type of clothing that adheres to the body 1, and has such a composition that the resistance lining part 3 always adheres to the skin surface, for example. The aforementioned operations/effects are thereby demonstrated with significant effectiveness.

For example, a configuration is adopted in which the aforementioned garment body 2 that adheres to the body 1 is used, the aforementioned resistance lining part 3 is formed on an internal surface of the garment body 2, and the position at which the resistance lining part 3 adheres to the skin surface of the body 1 does not move in relation to the skin surface as easily as the other internal surfaces. Satisfactory taping effects are thereby obtained in any position from the resistance lining part 3, and the aforementioned operations/effects are demonstrated with significant effectiveness.

Although the resistance lining part 3 may not necessarily be elastic, when an elastic material is used to form the resistance lining part 3, the resistance lining part 3 demonstrates the aforementioned taping effects while not compromising the ease of movement of the body 1 on which the sports garment is worn, and an environment that enables significant ease of movement is obtained.

Furthermore, when, for example, the garment body 2 and the resistance lining part 3 are endowed with elasticity, and the resistance lining part 3 has less elasticity than the garment body 2, the resistance lining part 3 does not stretch in the same manner as the garment body 2 even when the resistance lining part 3 is elastic. The sports garment therefore has an extremely effective structure that reliably supports muscles and other parts and demonstrates the aforementioned taping effects while maintaining ease of movement.

When, for example, the resistance lining part 3 is bonded and fixed to the internal surface of the garment body 2, it is extremely easy to form the resistance lining part 3 with the garment body 2, and when, for example, the resistance lining part 3 is formed in the garment body 2 by sewing, there is a difference in elasticity between the sewn regions and the garment body 2 during exercise, and muscles and other parts can become severely compressed. However, the configuration in which the resistance lining part 3 is bonded reduces the likelihood of an excessive difference in elasticity such as occurs when the resistance lining part 3 and the garment body 2 are sewn together. The resistance lining part 3 therefore evenly supports the muscles and other parts with a uniform pressure, and since the use of a bonded structure minimizes the number of sewn regions in the garment body 2, contact between stitches and the skin surface is reduced and easier movement and enhanced comfort can be provided.

### Example 1

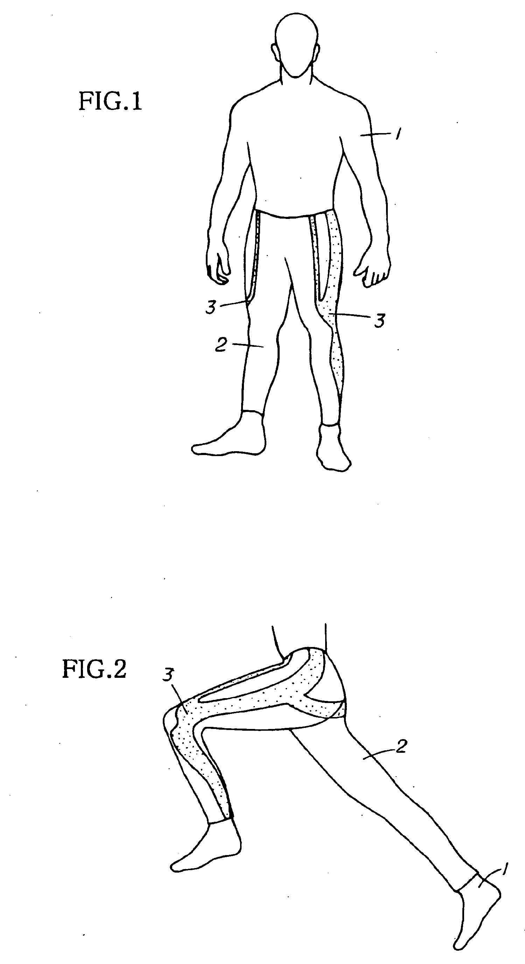

A first specific example of the present invention will be described based on FIGS. 1 and 2.

In this example, the present invention is applied to long-pant-type leggings as the garment body 2 that extend from the waist to the ankles.

Specifically, the garment body 2 is formed from an elastic material, and is formed so as to be able to fit the body 1 (lower half of the body) precisely, to suppress unnecessary vibration of muscles, and to reduce fatigue.

The garment body 2 is sewn and formed from a material that has minimal frictional resistance with respect to the skin surface of the body 1, i.e., a material that easily slides against the skin.

In this example, the resistance lining parts 3 are formed on the internal surface of the garment body 2 by using a material that has high frictional resistance and does not easily slide with respect to the skin surface of the body 1.

Specifically, the resistance lining parts 3 are formed using a belt-shaped, linear material, the surface of the material is raised, and frictional resistance with the skin surface is increased by the piles.

The resulting resistance lining parts 3 having irregular surfaces may be formed by the stitching (weaving) structure, for example, and frictional resistance with the skin surface may be increased by the surface irregularity of the resistance lining parts 3.

In the present example, the resistance lining parts 3 are bonded and fixed to the internal surface of the garment body 2 by an adhesive.

The resistance lining parts 3 are also formed in a direction whereby the same effects are obtained as a taping effect in which tape is wrapped onto the body 1 to restrict movement of muscles, joints, and other parts so as to prevent contusions, bruising, muscle strain, sprain, bone fracture, and other injury.

According to the taping theory in sports science, tape is applied or wrapped to restrict movement of joints, muscles, or the like and to reinforce joints, muscles, and other parts, or the tape is applied to prevent unnecessary force from being applied to the taped parts. This theory was researched in order to protect joints, ligaments, muscles, bones, and other parts of the body 1 from the legs, arms, and hips to the shoulders, neck, and fingers, and to prevent the occurrence and recurrence of contusions, bruising, muscle strain, sprain, fracture, and other sports injuries.

In the present example, a design is adopted so that the region from the waist to the calves in particular is protected according to the aforementioned taping theory.

Specifically, based on this theory, the resistance lining parts 3 having a large amount of frictional resistance with respect to the skin are formed in meandering fashion in the length direction of the lower body so as to extend from the wearer's waist to the buttocks, hips, knees, and calves.

Furthermore, one or both of the band-shaped and/or linear resistance lining parts 3 slightly curved along the length direction of the muscle fibers of the waist, buttocks, hips, knees, and calves of the wearer are formed so as to extend from the upper edge of the waist part of the internal surface of the garment body 2 to the bottom (lower) ends of the calves. A configuration is adopted that is based on the theory that joint protection and protection of muscles from the waist to the buttocks, hips, knees, and calves can be anticipated when one of the resistance lining parts 3 comes in contact with (slides against) the skin surface and restricts movement of the skin in the taping direction, and this protection makes it possible to prevent the occurrence or recurrence of muscle strain in the lower body, contusions of the hip joints and knee joints, and other sports injuries. The drawings show a case in which the resistance lining parts 3 are formed on the outside parts of the body 1 in the foot parts in particular.

Accordingly, the resistance lining parts 3 that are set with consideration for the movement of the muscles of the lower body, or the movement of the hip and knee joints, are configured so as to prevent contusions, muscle strain, or the like, as well as to enhance the fit on the body 1 and even further reduce fatigue during exercise.

The resistance lining parts 3 in the present example are also elastic, and the elasticity of the resistance lining parts 3 is set so as to be less than the elasticity of the garment body 2.

Specifically, the resistance lining parts 3 are formed using an elastic material, and the elastic material used for the resistance lining parts 3 has less elasticity than the elastic material used for the garment body 2.

A configuration may also be adopted in which the elasticity of the resistance lining parts 3 is made less than the elasticity of the garment body 2 by a technique in which the resistance lining parts 3 are formed by stacking two layers of material that have the same elasticity as the garment body 2.

Accordingly, the resistance lining parts 3 are elastic. However, the resistance lining parts 3 do not stretch to the same degree as the garment body 2 but demonstrate appropriate elasticity, reliably support muscles and other parts, and produce the aforementioned taping effects while ensuring ease of movement.

Since the skin also follows the stretching of the garment body 2 even though a large frictional resistance is imparted to the skin surface by the resistance lining parts 3 during exercise, an environment allows easy movement (easy movement of the body). As a result, effects are demonstrated whereby the range in which muscles and tendons can move is increased within an appropriate range.

Specifically, only the skin surfaces of muscles, tendons, or other certain parts move due to garment wearing and exercise, and internal tissues can easily move without the skin itself continuing to press on the muscles and other parts.

In the present example, a case was described in which the present invention was applied to a pant-type garment body 2, but the present invention may also be applied to a jacket-type garment body 2 to impart the taping effects to the arms, shoulders, and other parts.

### Example 2

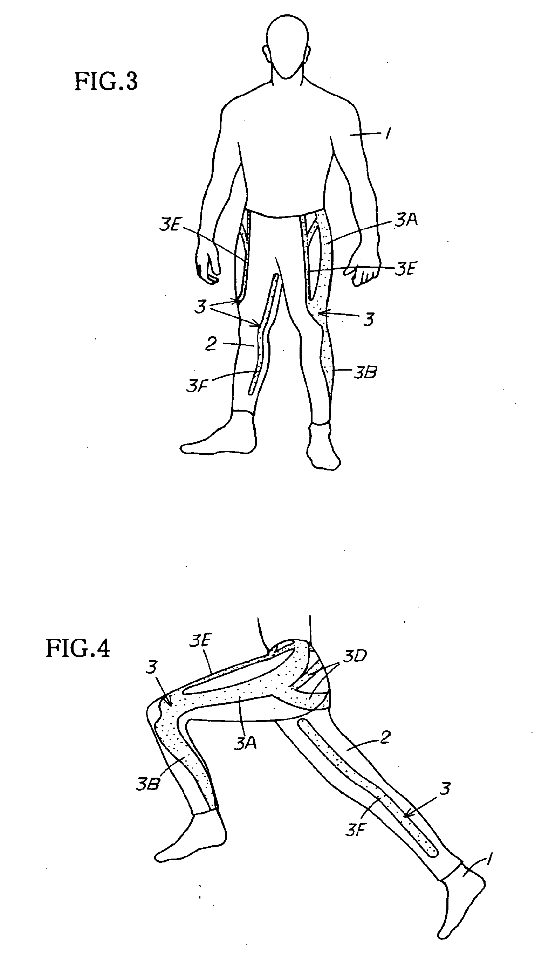

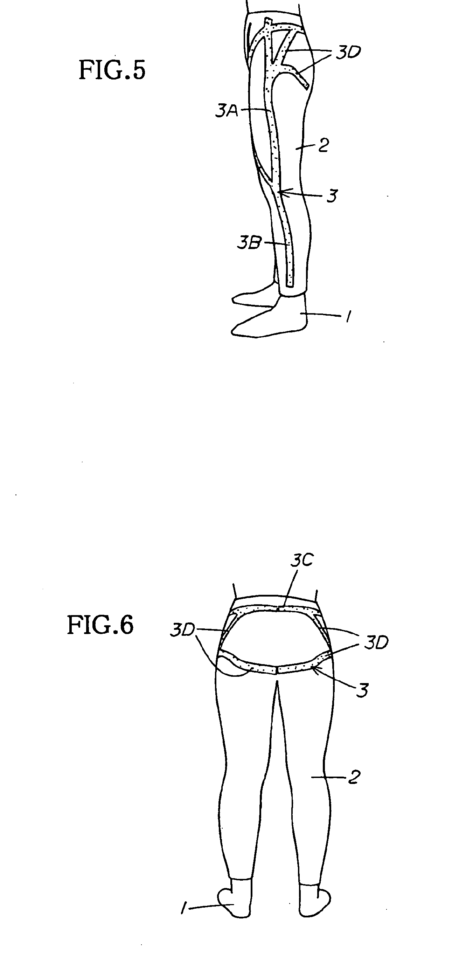

A second specific example of the present invention will be described based on FIGS. 3 through 6.

The present example is of a case in which a larger number of the resistance lining parts 3 in Example 1 is added to achieve a wide range of muscle protection or prevention of the recurrence of sports injuries.

Specifically, the resistance lining parts 3 of the present example are composed of a tensor fascia lata muscle lining 3A, a tibialis anterior muscle lining 3B, an L4-5 lining 3C, gluteus medius linings 3D, a quadriceps femoris muscle lining 3E, and a gracilis muscle lining 3F.

The tensor fascia lata muscle lining 3A is formed on the outside of the upper thigh, and the lower portion is formed along the tensor fascia lata muscle that transfers to the iliotibial tract and extends to the outside of the knee. The tensor fascia lata muscle assists in bending and rotating the hips, extending the knee joints, and other movements. Force is applied to this area during sprinting and other forceful stepping with the legs, and the leg stamina is reduced when this muscle is injured. The tensor fascia lata muscle lining 3A is capable of preventing the occurrence or recurrence of sports injury to the tensor fascia lata muscle.

The tibialis anterior muscle lining 3B is formed at the muscle on the outside of the shin of the lower leg (portion below the knee), and along the tibialis anterior muscle that extends from below the knee to the front of the sole of the foot. The tibialis anterior muscle raises and rotates the foot inward. This muscle is used for kicking out and landing during running, and is always in a tensed state when the body is in a forward-bent posture during skiing. A sprain can easily occur when this muscle is fatigued. The tibialis anterior muscle lining 3B is capable of preventing the occurrence or recurrence of sports injury to the tibialis anterior muscle.

The L4-5 (L is an abbreviation for "lumbar," and 4 and 5 are the fourth and fifth of five lumbar vertebrae) lining 3C is a transversely directed lining formed so as to pass through positions that correspond to the fourth and fifth lumbar vertebrae. The lumbar vertebrae control numerous actions in exercise, such as vibration due to jumping, sudden stops, and rapid twisting. The L4-5 lining 3C is capable of preventing the occurrence or recurrence of sports injury to the lumbar vertebrae.

The gluteus medius linings 3D reduce the burden on the gluteus medius muscles and are formed in a layer below the gluteus maximus muscles and in a position along the gluteus medius muscles somewhat above the gluteus maximus muscles. The gluteus medius muscles stabilize the movement of the hip joints. These muscles are necessary for balance during walking or exercise, and when these muscles are weakened, the pelvis and thigh muscles lose stability and are easily affected during sports that require balance in a crouching position, such as sumo wrestling, wrestling, and baseball defense. The gluteus medius linings 3D are capable of preventing the occurrence or recurrence of sports injury to the gluteus medius muscles.

The quadriceps femoris muscle linings 3E are formed along the quadriceps femoris muscles that are composed of four muscle heads at the front of the thighs, i.e., the rectus femoris muscle, the medial vastus muscle, the lateral vastus muscle, and the intermediate vastus muscle. The quadriceps femoris muscles provide significant power to extension of the knees. These muscles are in the thigh region that is most important in sports, and injury to the knees easily occurs when the quadriceps femoris muscles are fatigued during sports in which a large burden is placed on the legs, such as in track and field events, soccer, volleyball, basketball, and skiing. The quadriceps femoris muscle linings 3E are capable of preventing the occurrence or recurrence of sports injury to the quadriceps femoris muscles.

The gracilis muscle linings 3F are formed inside the thighs, along the thin muscle, i.e., gracilis muscle, extending from the crotch to below the knee. The gracilis muscle assists in inward rotation of the leg and the hip joint. The gracilis muscle serves to prevent the knee from turning inward due to fatigue. The gracilis muscle linings 3F are capable of preventing the occurrence or recurrence of sports injury to the gracilis muscle.

### Example 3

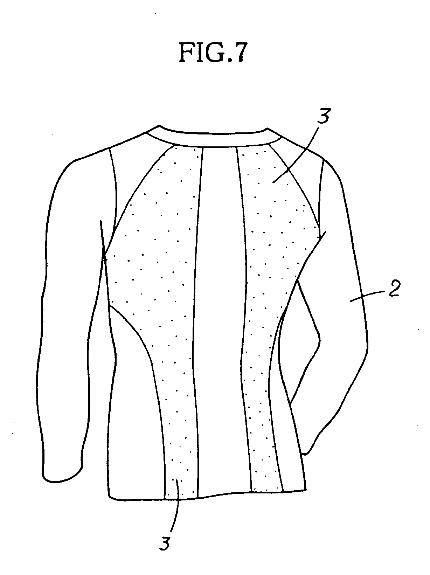

A third specific example of the present invention will be described based on FIG. 7.

In the present example, the present invention is applied to a long-sleeve jacket as the garment body 2 that is designed so that the back area in particular is protected according to the taping theory of Example 1.

Specifically, the resistance lining parts 3 having high frictional resistance with respect to the skin are formed in the length direction of the upper body so as to extend from the upper back of the wearer to the lower back.

Specifically, belt-shaped resistance lining parts 3 adjacent to the shoulder blades and latissimus dorsi muscles of the back are formed on the left and right portions other than the spine portion so as to extend from the upper edge of the back to the bottom (lower) edge of the back of the internal surface of the rear side of the garment body 2. A configuration is adopted that is based on the theory that shoulder joint protection and protection of muscles in the back can be anticipated when the resistance lining parts 3 come in contact with (slide against) the skin surface and restrict movement of the skin in the taping direction, and this protection makes it possible to prevent the occurrence or recurrence of muscle strain in the upper body, contusions of the shoulder joints, and other sports injuries.

Effects are also anticipated whereby the dorsal muscles are extended and posture is corrected, and enhanced ease of arm muscle extension and breathing stability during exercise are obtained. Since the arms also move about the shoulder blades, a state is created in which the arms move easily, and exercise is facilitated through the taping effects that allow the arms to move properly.

In the present example, a configuration is adopted in which the resistance lining parts 3 are provided to areas other than the spine portion, whereby the spine portion extends more easily than the portions in which the resistance lining parts 3 are present. The conforming properties of the garment with respect to exercises such as twisting and forward-backward and side-to-side movement thereby remain the same, and heat-releasing effects can be anticipated in parts where more perspiration occurs and accumulates more easily.

Accordingly, contusions, muscle strain, or other injuries are prevented, the fit to the body 1 is enhanced, and exercise fatigue is even further reduced by resistance lining parts 3 that are designed with consideration for the movement of upper body muscles and shoulder joints.

## Claims

1. A sports garment **characterized in that** a garment body (2) for adhering to a body (1) is formed using a material that has low frictional resistance and slides easily against the skin surface of a body (1);
a resistance lining part (3) is formed on the internal surface of the garment body (2) by using a material that has high frictional resistance and does not easily slide against the skin surface of a body (1) ;
a position at which the resistance lining part (3) adheres to a skin surface of the body (1) varies less easily than that of another adhering internal surface of the garment body (2);
said garment body (2) and said resistance lining part (3) are formed so as to be elastic, and an elasticity of the resistance lining part (3) is set so as to be less than an elasticity of the garment body (2); and
the resistance lining part (3) is formed in a direction whereby the same effects are obtained as a taping effect in which tape is wrapped onto a body (1) to restrict movement of muscles, joints, and other parts so as to prevent contusions, bruising, muscle strain, sprain, bone fracture, and other injury.

2. The sports garment according to claim 1, **characterized in that** said resistance lining part (3) is bonded and fixed to an internal surface of said garment body (2).

## Patentansprüche

1. Sportbekleidung, **dadurch gekennzeichnet, dass** ein Bekleidungskörper (2) zum Anliegen an einem Körper (1) verwendend ein Material mit einem niedrigen Reibungswiderstand gebildet ist und leicht auf der Hautoberfläche eines Körpers (1) gleitet,
ein Widerstandsausrichtabschnitt (3) auf der inneren Oberfläche des Bekleidungskörpers (2) verwendend ein Material, das einen hohen Reibwert hat und nicht leicht über die Hautoberfläche eines Körpers (1) gleitet, geformt ist;
ein Ort an dem der Widerstandsausrichtabschnitt (3) an einer Hautoberfläche des Körpers (1) anhaftet, weniger leicht variiert als der von einer anderen anhaftenden inneren Oberfläche des Bekleidungskörpers (2);
wobei der Bekleidungskörper (2) und der Widerstandsausrichtabschnitt (3) geformt sind, um elastisch zu sein und eine Elastizität des Widerstandsausrichtabschnitts (3) eingestellt ist, um geringer zu sein als eine Elastizität des Bekleidungskörpers (2); und
wobei der Widerstandsausrichtabschnitt (3) in einer Richtung geformt ist, wodurch die gleichen Effekte erhalten werden wie bei einem Bandumwicklungseffekt bei dem ein Band auf einen Körper (1) gewickelt wird, um die Bewegung von Muskeln, Gelenken, und anderen Teilen zu begrenzen, um Prellungen, blaue Flecken, Muskeldehnungen, Verstauchungen, Knochenfrakturen, und andere Verletzungen zu verhindern.

2. Sportbekleidung nach Anspruch 1, **dadurch gekennzeichnet, dass** der Widerstandsausrichtabschnitt (3) an einer inneren Oberfläche des Bekleidungskörpers (2) gebondet und fixiert ist.

## Revendications

1. Vêtement de sport **caractérisé en ce qu'**un élément de vêtement (2) destiné à adhérer à un corps (1) est formé en utilisant un matériau qui a une faible résistance au frottement et glisse facilement contre la surface de la peau d'un corps (1) ;
une partie de doublure résistante (3) est formée sur la surface interne de l'élément de vêtement (2) en utilisant un matériau qui a une forte résistance au frottement et qui ne glisse pas facilement contre la surface de la peau d'un corps (1) ;
une position à laquelle la partie de doublure résistante (3) adhère à une surface de la peau du corps (1) varie moins facilement que celle d'une autre surface interne adhérente de l'élément de vêtement (2) ;
ledit élément de vêtement (2) et ladite partie de doublure résistante (3) sont formés de manière à être élastiques, et une élasticité de la partie de doublure résistante (3) est établie de manière à être inférieure à une élasticité de l'élément de vêtement (2) ; et
la partie de doublure résistante (3) est formée dans une direction dans laquelle les mêmes effets sont obtenus qu'un effet de bandage dans lequel une bande est enroulée autour d'un corps (1) pour limiter un mouvement des muscles, des articulations, et d'autres parties de manière à empêcher des ecchymoses, des contusions, une élongation, une entorse, une fracture d'un os et d'autres blessures.

2. Vêtement de sport selon la revendication 1, **caractérisé en ce que** ladite partie de doublure résistante (3) est liée et fixée à une surface interne dudit élément de vêtement (2).
